Europäisches Patentamt

(19) European Patent Office　　(11) Numéro de publication: **0 022 005**

Office européen des brevets　　　　　　　　　　　　**B1**

⑫　　FASCICULE DE BREVET EUROPEEN

(45) Date de publication du fascicule du brevet: **17.04.85**　(51) Int. Cl.⁴: **G 01 N 33/538,** C 12 Q 1/00,
G 01 N 33/74

(21) Numéro de dépôt: **80400913.2**

(22) Date de dépôt: **19.06.80**

(54) Procédé de séparation d'une substance protéinique à partir d'une solution la contenant par filtration d'affinité et application dudit procédé aux dosages enzymatiques.

(30) Priorité: **21.06.79 FR 7915992**

(43) Date de publication de la demande:
**07.01.81 Bulletin 81/01**

(45) Mention de la délivrance du brevet:
**17.04.85 Bulletin 85/16**

(84) Etats contractants désignés:
**AT BE CH DE FR GB IT LI LU NL SE**

(56) Documents cités:
**DE-A-2 719 772**
**FR-A-2 074 027**
**FR-A-2 184 054**
**FR-A-2 379 552**
**US-A-3 502 545**

**JOURNAL OF CHROMOTOGRAPHY; vol. 171, 1 avril 1979 Amsterdam, NL A. CHAABOUNI et al.: "Affinity Partition of proteins in aqueous two-phase systems containing polyoxyethylene glycol-bound ligand and charged dextrans", pages 135-143**

(73) Titulaire: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Nicolas, Jean-Claude**
***Le Phaeton avenue du Parc Saulas***
**F-34000 Montpellier (FR)**
Inventeur: **Terouanne, Béatrice**
**10 Rue Alauzet**
**F-34000 Montpellier (FR)**
Inventeur: **Descomps, Bernard**
**26 Avenue d'Anas**
**F-34000 Montpellier (FR)**
Inventeur: **Crastes de Paulet, André**
**12 Avenue Lepic**
**F-34000 Montpellier (FR)**

(74) Mandataire: **Harlé, Robert et al**
**c/o Cabinet Harlé & Phélip 21, rue de la Rochefoucauld**
**F-75009 Paris (FR)**

(56) Documents cités:
**SNYDER-KIRKLAND: "Introduction to modern liquid chromatography John Wiley & Sons 1979 pages 490 fig. 12.2 et 493, 494**

**EP 0 022 005 B1**

Courier Press, Leamington Spa, England.

# 0 022 005

**Description**

La présente invention concerne l'application de la séparation sélective de substances protéiniques à partir d'une solution les contenant par filtration d'affinité, aux dosages enzymatiques faisant intervenir un agent de liaison et une substance marquée par une enzyme, tels que les dosages immunoenzymatiques.

Plus précisément, la présente invention concerne l'application aux dosages enzymatiques de la séparation sélective d'une substance protéinique donnée d'autres substances à poids moléculaire élevé, supérieur à celui de ladite substance protéinique, donée, lesdites substances étant en solution, par exemple dans un liquide biologique.

On connaît déjà des procédés de séparation de substances protéiniques, tels que par exemple la séparation par filtration sur un gel, ou la chromatographie, telle que la chromatographie d'affinité. Ces procédés, bien connus de l'homme de l'art, présentent copendant des inconvénients. Par exemple, la filtration sur gel permet d'obtenir un filtrat contenant les substances exclues par le gel, c'est-à-dire les substances ayant un poids moléculaire supérieur à une valeur donnée, qui est fonction de la nature et de la structure du gel, tandis que le gel retient toutes les substances ayant un poids moléculaire inférieur à la valeur donnée en cause. Toutefois, ce procédé ne permet pas une séparation sélective d'une substance protéinique déterminée.

Un autre procédé pour séparer des substances protéiniques est la chromatographie sur gel. Ce procédé nécessite souvent la mise en oeuvre de colonnes de gels de longueur importante et des temps d'élution relativement longs, ce qui empêche l'obtention d'une séparation rapide souvent nécessaire dans le domaine biologique, par exemple dans les dosages immunologiques. Le chromatographie d'affinité, souvent utilisée pour la purification d'enzymes ou la préparation de conjugés antigène-enzyme (Exley D. and Abukneska, 1977, FEBS letters 79 p. 301—304 et Lanner et al. (1977) Proceedings of an International Symposium, Vienne) ne permet pas cependant de séparer deux substances ayant la même affinité pour le gel, mais dont les poids moléculaires sont différents.

A titre d'exemples de documents concernant de tels procédés de séparation on citera:

— le brevet US 3502.545 qui concerne un procédé pour la séparation de substances polymères solubles dans l'eau à partir de protéines ou peptides non liés en utilisant un gel semi-poreux.

Ce procédé met en oeuvre une colonne de chromatographie contenant un gel semi-poreux ayant des limites d'exclusion supérieures au poids moléculaire du polymère soluble dans l'eau et supérieures au poids moléculaire de la protéine ou peptide soluble dans l'eau non lié.

— la demande de brevet FR—A—2379552 qui concerne une matrice activée et une méthode d'activation pour chromatographie d'affinité. L'activation consiste en une carbonylation d'un polysaccharide avec un agent de carbonylation approprié. Ce document concerne donc uniquement des supports pour chromatographie d'affinité appropriés pour les procédés de séparation faisant intervenir une telle chromatographie d'affinité.

— le brevet FR—A—2074027 concerne un procédé pour le couplage de polymères avec des molécules biologiquement actives. Ce procédé consiste à coupler à l'aide de ponts covalents deux substances ou plus, dont l'une au moins est un polymère servant de support, chacune des substances contenant au moins l'un des groupes fonctionnels suivants: isonitrile, aldéhyde ou cétone, anion, amine primaire ou secondaire, la réaction de couplage ayant lieu dans un mélange réactionnel contenant les substances spécifiées, avec éventuellement des substances additionnelles, de manière que tous les groupes fonctionnels en question soient présents simultanément au début de la réaction.

Ces produits de couplage obtenus selon ce procédé conviennent comme supports pour chromatographie d'affinité.

— le brevet FR—A—2184054 qui concerne un procédé de séparation de composés biologiquement actifs par chromatographie d'affinité sur absorbants sélectifs.

On utilise selon l'invention un procédé de séparation selective d'au moins une substance protéinique présentant une affinité pour un ligand, d'autres substances parmi lesquelles certaines ont la même affinité vis-à-vis dudit ligand mais un poids moléculaire élevé, supérieur à celui de ladite substance protéinique, lesdites substances étant en solution, ledit procédé consistant à filtrer la solution contenant lesdites substances sur un gel excluant lesdites substances à poids moléculaire élevé, ledit gel étant couplé avec un ligand pour lequel ladite substance protéinique présente une affinité.

Ce procédé permet donc de séparer sélectivement au moins une substance protéinique donnée, présentant une affinité pour un ligand, de substances parmi lesquelles certaines ont la même affinité pour le ligand mais un poids moléculaire élevé, supérieur à celui de ladite substance protéinique. En effet, en raison du choix du gel, les substances à poids moléculaire élevé, supérieur à celui de ladite substance protéinique, seront exclues, qu'elles présentent ou non une affinité pour le ligand. Les autres substances éventuellement présentes dans la solution et susceptibles de pénétrer dans le réseau interne du gel en

2

# 0 022 005

raison de leur poids moléculaire ne seront pas sélectivement retenues par celui-ci, dans la mesure où elles ne possèdent pas d'affinité pour le ligand.

La récupération de la substance protéinique donnée après la filtration d'affinité peut être effectuée après lavage du gel, par élution selon des moyens appropriés connus de l'homme de l'art, par exemple avec un changement de pH ou par compétition avec un excès de ligand en solution.

Il faut noter que dans l'application dudit procédé, c'est-à-dire dans les dosages enzymatiques, la récupération de ladite substance protéinique n'est pas nécessaire puisque la mesure de l'activité enzymatique est effectuée sur le filtrat comme cela sera explicité ci-après.

Le gel mis en oeuvre dans le procédé selon la présente invention doit être un gel réticulé, de telle sorte qu'il exclut les substances de poids moléculaire élevé, supérieur à celui de la substance protéinique que l'on désire séparer. En d'autres termes, le gel mis en oeuvre dans le procédé utilisé selon l'invention doit avoir une dimension de pores telle que le gel puisse exclure les substances à poids moléculaire élevé, supérieur à celui de ladite substance protéinique considérée.

A titre d'exemples de gels qui conviennent aux fins de l'invention, on peut citer les gels de polyacrylamide, d'agarose, de polyacrylamide-agarose, de dextrane, de polysaccharides etc, en particulier les gels connus sous les dénominations commerciales ULTROGEL, SEPHADEX, BIORAD et SEPHAROSE.

Le gel mis en oeuvre selon ledit procédé est couplé avec un ligand pour lequel la substance protéinique à séparer présente une affinité. Le couplage gel-ligand est réalisé selon des procédés connus, par exemple par activation du gel à l'aide de glutaraldéhyde, de bromure de cyanogène, de bis-époxy-des et couplage avec le ligand selon des procédés à la portée de l'homme de l'art. A cet effet, on pourra citer, à titre d'ouvrage de référence, Methods in Enzymology- vol. 34, pages 13 à 102.

La quantité de ligand couplé avec le gel dépend de l'affinité de la substance protéinique considérée pour le ligand. On a trouvé qu'une quantité d'enivron 25 à 200 fois, par exemple de 50 fois, la constante d'association de la substance protéinique pour le ligand, était appropriée aux fins de l'invention.

Au sens de la présente invention, on désigne par l'expression "substance protéinique" aussi bien les protéines elles-mêmes telles que les enzymes, les anticorps, les antigènes et similaires, que des conjugués protéiniques, tels que par exemple les substances marquées par une enzyme utilisées dans les dosages enzymatiques.

Selon l'invention des dosages enzymatiques faisant intervenir un agent de liaison et une substance marquée par une enzyme utilisent ce procédé de séparation. A titre d'exemple de tels dosages enzymatiques, on citera notamment les dosages immunoenzymatiques.

Les progrès importants accomplis dans l'enzymologie ont permis de substituer aux dosages radioimmunologiques des dosages immunoenzymatiques faisant appel à une enzyme comme marqueur, au lieu des marqueurs radioactifs utilisés jusqu'alors. Il existe de nombreuses références bibliographiques dans le domaine des dosages immunoenzymatiques. On pourra par exemple se référer à l'article de S. AVRAMEAS "Détection d'anticorps et d'antigènes à l'aide d'enzymes" Bull. Soc. Chim. Biol. 1968, *50* n° 5—6.

On rappellera que les dosages enzymatiques, tels que les dosages immunoenzymatiques, peuvent être classés en deux catégories, à savoir les dosages directs d'une part et les dosages par compétition d'autre part. Par ailleurs, il existe différents moyens pour séparer la substance marquée par une enzyme n'ayant pas réagi des complexes formés au cours du dosage. Un exemple de ces moyens consiste en l'utilisation d'un des réactifs de dosage sous une forme insolubilisée. A cet effet, on peut citer notamment la technique bien connue de dosage par immunoadsorbant. Un composé immunochimique particulier qui convient comme immunoadsorbent est décrit dans la demande de brevet DE—A—2.719.772.

Un autre moyen, également bien connu dans le domaine immunoenzymatique, est la technique dite de dosage par double anticorps, qui consiste à faire précipiter le complexe formé au cours du dosage à l'aide d'un anticorps dudit complexe.

Les procédés nécessitant la mise en oeuvre d'un immunoadsorbant sont dits "hétérogènes". Ces procédés impliquent d'une part la préparation d'un tel immunoadsorbant, qui ne peut être utilisé qu'une fois, et d'autre part un certain nombre de lavages et de centrifugations pour séparer l'immunoadsorbant du milieu réactionnel. En effet, il est préférable de déterminer l'activité enzymatique de la fraction liée à l'immunoadsorbent afin d'avoir un dosage plus précis. Il est donc difficile d'automatiser ces procédés de dosages enzymatiques.

On donnera ci-après quelques exemples de dosages immunoenzymatiques mettant en oeuvre des immunoadsorbants dans le cas de dosages d'antigènes. A cet effet, on désignera par conjugué anticorps (antigène)-enzyme" l'anticorps ou l'antigène marqué par une enzyme, et par "conjugué anticorps (antigène)-enzyme libre" le conjugué anticorps (antigène)-enzyme n'ayant pas reagi au cours du dosage immunoenzymatique.

Un procédé de dosage immunoenzymatique d'antigène bien connu consiste:

— à faire incuber l'antigène à doser avec un conjugué anticorps-enzyme,
— à ajouter au milieu réactionnel résultant un antigène insolubilisé, par exemple un antigène fixé sur un immunoadsorbant qui réagit avec le conjugué anticorps-enzyme libre,
— à séparer la phase solide de la phase liquide et à mesurer l'activité enzymatique de l'une des phases.

3

**0 022 005**

Le procédé de dosage immunoenzymatique d'antigènes par compétition consiste:

— à faire incuber les antigènes à tester avec un conjugué antigène-enzyme et un anticorps, mettant ainsi en oeuvre la compétition de l'antigène à doser et du conjugué antigène-enzyme vis-à-vis de l'anticorps, celui-ci étant sous une forme insoluble, par exemple fixé sur un immunoadsorbant,
— à séparer la phase liquide contenant le conjugué antigène-enzyme libre de la phase solide constituée du complexe antigène-enzyme/anticorps, et
— à mesurer l'activité enzymatique de l'une des deux phases.

Un autre procédé de dosage immunoenzymatique pour le dosage des antigènes est le dosage par double anticorps qui consiste:

— à faire incuber l'antigène à doser avec une quantité déterminée d'un conjugué antigène-enzyme et d'un anticorps $Ac_1$,
— à faire ensuite incuber le mélange réactionnel résultant avec un anticorps $Ac_2$, anticorps du complexe antigène-enzyme formé lors de la première étape, l'anticorps $Ac_2$ étant fixé sur un immunoadsorbant,
— à séparer la phase liquide de la phase solide, et à mesurer l'activité enzymatique de l'une des deux phases.

Les procédés de dosage décrits ci-dessus, qui peuvent également être utilisés pour le dosage d'anticorps, sont donnés à titre illustratif de procédé impliquant le mise en oeuvre d'un immunoadsorbant.

Ces exemples montrent qu'une étape de séparation phase solide/phase liquide est toujours nécessaire, celle-ci impliquant des lavages et centrifugations qui empêchent l'automatisation desdits procédés de dosages immunoenzymatiques. De plus, comme on l'a indiqué précédemment, l'immunoadsorbant ne peut être utilisé qu'une seule fois, ce qui exige la mise en oeuvre de quantités importantes de support insoluble et implique des réactions de couplage support/antigène (anticorps) pour fabriquer l'immunoadsorbant approprié.

Le procédé de séparation précédemment décrit est, selon l'invention, appliqué aux procédés de dosages enzymatiques faisant intervenir un agent de liaison et une substance marquée par une enzyme pour la séparation de la substance marquée par une enzyme n'ayant pas réagi des complexes formés au cours du dosage. Par l'expression "complexes formés au cours du dosage" on désigne le ou les produits de réaction d'au moins un agent de liaison avec une substance marquée par une enzyme, qui se forment au cours du dosage enzymatique selon le type de dosage choisi, par exemple dosage direct, dosage par compétition, dosage sandwich et dosage à double anticorps.

Le procédé de séparation utilisé selon l'invention évite l'utilisation d'immunoadsorbant et donc les étapes de lavage et de centrifugation citées ci-dessus. De ce fait, les dosages enzymatiques, tels que les dosages immunoenzymatiques, sont réalisés plus rapidement et peuvent, ce qui présente un intérêt pratique considérable, être automatisés.

La présente invention concerne donc un procédé de dosage enzymatique, direct ou par compétition, des produits de réaction d'au moins un agent de liaison avec une substance marquée par une enzyme, ledit agent de liaison ayant une affinité vis-à-vis de ladite substance marquée par une enzyme, consistant à faire réagir ledit agent de liaison à doser avec ladite substance marquée par une enzyme lorsqu'il s'agit d'un dosage direct, ou ledit agent de liaison avec ladite substance marquée par une enzyme et ladite substance à doser lorsqu'il s'agit d'un dosage par compétition, caractérisé en ce qu'il consiste à séparer ladite substance marquée par une enzyme n'ayant pas réagi dudit ou desdits produits de réactions, par filtration d'affinité, de la solution obtenue après réaction, sur un gel couplé avec un ligand pour lequel l'enzyme possède une affinité, ledit gel ayant des pores d'une taille suffisante pour exclure le ou lesdits produits de la réaction de poids moléculaire élevé et supérieur à celui de la substance marquée par une enzyme et à mesurer, de façon connue, l'activité enzymatique de la solution ainsi filtrée.

A titre d'exemples d'agents de liaison qui peuvent être mis en oeuvre selon l'invention, on peut citer les protéines de liaison, telles que les anticorps, les protéines de transport, par exemple la transcortine, les récepteur hormonaux et similaires. On pourra également considérer comme agents de liaison les antigènes polyfonctionnels ou de poids moléculaire élevé; dans ce cas, le ligand de l'antigène sera l'anticorps spécifique. A titre d'exemples de tels antigènes, on citera les polypeptides, polysaccharides et polynucléotides.

La substance marquée par une enzyme est une substance quelconque présentant une affinité vis-à-vis de l'agent de liaison, c'est-à-dire pouvant être considérée comme le ligand de l'agent de liaison.

L'agent de liaison constitue soit le réactif de dosage lorsqu'il s'agit d'un dosage par compétition, soit la substance à doser lorsqu'il s'agit d'un dosage direct.

A titre d'illustration, on a indiqué dans le tableau I différents exemples de dosages enzymatiques qui peuvent être réalisés selon la procédé de l'invention.

Le gel mis en oeuvre dans le procédé de dosage enzymatique selon l'invention est tel que défini ci-dessus et peut être choisi parmi les gels de polyacrylamide, d'agarose, de polyacrylamide-agarose, de polysaccharide et de dextrane, pourvu qu'il ait des pores d'une taille suffisante pour exclure le ou lesdits produits de la réaction de poids moléculaire élevé et supérieur à celui de la substance marquée par une

4

enzyme. De plus, le gel doit être couplé avec un ligand vis-à-vis duquel l'enzyme présente une affinité. Ces ligands sont choisis parmi les inhibiteurs d'enzyme, les co-enzymes et les substrats d'enzyme. Il sera aisé à l'homme de l'art de déterminer le ligand à utiliser en fonction de l'enzyme mise en oeuvre et choisir le mode de couplage approprié en fonction du ligand et du gel choisi. Toutefois, ou pourra utilement se référer à l'article "Methods in Enzymology" vol. 34, p. 13—102, dans lequel sont décrits les principaux modes de couplage.

A titre d'exemples, on indiquera que lorsque l'enzyme est une déshydrogénase à NAD(P) (c'est-à-dire une enzyme à nucléotides pyridiniques NAD du NADP), ou pourra utiliser comme ligand du NAD (nicotinamide-adénine-dinucléotide) du NADP (nicotinamide-adénine-dinucléotide-phosphate), de l'AMP (acide adénosine monophosphorique) ou de l'ADP (acide adénosine diphosphorique), qui sont des co-enzymes de deshydrogénases.

Les deshydrogénases qui peuvent être utilisées dans le procédé de l'invention sont des deshydrogénases de haute activité spécifique, telles que par exemple la glutathion réductase, la glucose 6-phosphate deshydrogénase, la malate-deshydrogénase, l'alcool deshydrogénase.

Lorsque l'enzyme est la $\Delta 5,3$-cétostéroïde isomérase, on pourra utiliser comme ligand un dérivé de l'oestradiol, l'oestradiol étant un inhibiteur de cette enzyme, tel que par exemple l'oestradiol-couplé en 17-$\beta$ au glutathion. Dans ce dernier cas, le couplage gel-ligand peut être réalisé par activation du gel avec du bromure de cyanogène, du glutaraldéhyde ou tout autre agent de couplage bien connu de l'homme de l'art.

Ainsi, le gel mis en oeuvre selon l'invention constitue un moyen de séparation approprié pour les dosages enzymatiques, qui permet, par simple filtration d'affinité, de retenir uniquement la substance marquée par une enzyme qui n'a pas réagi et d'obtenir un filtrat qui contient le ou les produits de la réaction, dont on mesure, de façon connue, l'activité enzymatique. Les procédés de dosages enzymatiques selon l'invention peuvent être de ce fait facilement automatisés, sur des appareils soit à flux continu, soit des appareils centrifuges. Cette automatisation peut être réalisée grâce à la rapidité de liaison entre le gel couplé avec un ligand et la substance marquée par une enzyme n'ayant pas réagi et à la possibilité d'une utilisation répétée puisqu'il suffit de laver le gel pour éliminer la substance marquée par une enzyme liée par affinité audit gel.

Le procédé de dosage enzymatique selon l'invention est particulièrement approprié pour les dosages d'antigènes et d'anticorps. Ainsi, par exemple, on pourra réaliser aisément selon l'invention le dosage d'un antigène, la substance marquée par une enzyme étant un conjugué anticorps-enzyme, l'anticorps étant spécifique de l'antigène; on obtient après l'incubation du liquide biologique contenant l'antigène à doser avec le conjugué anticorps-enzyme une solution contenant, d'une part, le conjugué anticorps-enzyme libre et, d'autre part, le complexe antigène/anticorps-enzyme. Le gel mis en oeuvre selon l'invention doit être tel qu'il exclut le complexe antigène/anticorps-enzyme et non le conjugué anticorps-enzyme, et comporter un ligand pour lequel l'enzyme possède une affinité; le conjugué anticorps-enzyme libre sera alors le seul retenu par le gel.

Selon une autre variante, le procédé de l'invention peut être appliqué aux dosages d'antigènes par compétition. Il y a lieu de mettre alors en oeuvre un conjugué antigène-enzyme comme substance marquée par une enzyme et un anticorps spécifique de l'antigène à doser, cet anticorps spécifique constituant l'agent de liaison. L'incubation du liquide biologique contenant l'antigène à doser avec le conjugué antigène-enzyme et l'anticorps spécifique conduit à la formation d'une solution contenant le conjugué antigène-enzyme libre, un complexe anticorps/antigène à doser et un complexe anticorps/antigène-enzyme. Le gel mis en oeuvre dans ce procédé pour séparer le conjugué antigène-enzyme libre des complexes doit avoir une dimension de pores telle qu'il exclut les complexes qui ont un poids moléculaire relativement élevé par rapport au conjugué antigène-enzyme libre et retient de façon sélective ledit conjugué grâce à la présence d'un ligand pour lequel l'enzyme présente une affinité.

Le marquage de la substance par une enzyme est réalisé selon des procédés bien connus de l'homme de l'art. A cet effet, on pourra se référer à l'article "Methods in Enzymology" vol. 25 part B pages 623 à 651.

On donnera ci-après des exemples spécifiques de dosages immunoenzymatiques selon l'invention mettant en oeuvre la $\Delta 5,3$-céto-stéroïde isomérase comme enzyme, le ligand couplé au gel étant l'oestradiol-glutathion. Dans ce cas particulier, le procédé de dosage immunoenzymatique selon l'invention peutêtre avantageusement mis en oeuvre pour le dosage d'antigènes constitués soit de substances de nature peptidique telles que les protéines, par exemple, l'alpha foetopropéine, les hormones peptidiques, par exemple l'hormone lactogène placentaire humaine HLP, soit de substances de nature stéroïdique, telles que les hormones stéroïdes, par exemple le progestérone.

Ainsi, une variante préférée du procédé de l'invention consiste en un procédé de dosage immunoenzymatique d'antigènes de nature peptidique ou stéroïdique contenus dans un liquide biologique, consistant à faire incuber lesdits antigènes à doser avec un conjugué antigène—$\Delta 5,3$ céto-stéroïde isomérase et des anticorps spécifiques desdits antigènes, utilisant comme réactif de dosage le conjugué progestérone—$\Delta 5,3$-céto-stéroïde isomérase ou le conjugué hormone lactogène placentaire—$\Delta 5,3$-céto-stéroïde-isomérase.

L'application de la $\Delta 5,3$-céto-stéroïde isomérase dans les dosages immunoenzymatiques est décrite en détail dans la demande de brevet FR—A—2432713 (EP—A—7879) au nom de la demanderesse, ainsi que les modes d'obtention des conjugués antigène—$\Delta 5,3$-céto-stéroïde isomérase.

**0 022 005**

On rappellera toutefois que le conjugué antigène-Δ5,3-céto-stéroïde isomérase, lorsque l'antigène est de nature peptidique, est réalisé par couplage par pont disulfure en faisant réagir d'une part la substance de nature peptidique et le 4-mercapto-butyrimidate de méthyle, et d'autre part la Δ5,3-cétostéroïde isomérase et l'anhydride 2-acétylmercaptosuccinique, et en couplant les produits respectifs de réaction, ce qui conduit à un conjugué dans lequel une mole d'enzyme est couplée à une mole de substance peptidique et qui conserve au moins 50% de son activité enzymatique après purification, celle-ci étant effectuée de manière connue par chromatographie d'affinité.

Lorsque l'antigène est de nature stéroïdique, le conjugué antigène-Δ5,3-céto-stéroïde isomérase est obtenu par couplage par l'intermédiaire d'un résidu thiol en faisant réagir d'une part la substance de nature stéroïdique avec l'acide bromoacétique de formule Br—CH$_2$—COOH, en présence de carbodiimide, et d'autre part la Δ5,3-céto-stéroïde isomérase avec l'anhydride S-acétyl-mercaptosuccinique suivie d'une désacétylation et en couplant les produits respectifs de réaction.

Dans ce cas particulier, on peut utiliser des gels qui excluent les substances ayant un poids moléculaire supérieur à 100.000. A titre d'exemples de gels appropriés, on citera les gels de polyacrylamide-agarose connus sous la dénomination commerciale "ULTROGEL" tels que les ULTROGEL AcA 34 et AcA 44, mis sur le marché par IBF (industrie Biologique Française).

Le procédé de l'invention convient aussi pour le dosage direct d'antigènes, tels que l'alphafoeto-protéine.

Dans ce cas, il y a lieu de former des conjugués anticorps-enzyme par des moyens appropriés, par exemple par couplage par ponts disulfure. Pour réaliser un tel couplage, on peut notamment faire réagir l'anticorps d'une part et l'enzyme d'autre part avec de l'anhydride S-acétylmercaptosuccinique et désacétyler puis coupler les produits respectifs de réaction. On a préparé ainsi un conjugué anticorps anti-alphafoetoprotéine-Δ5,3-céto-stéroïde isomérase.

Selon une variante de mise en oeuvre du procédé de l'invention, on peut utiliser en outre un anticorps de l'agent de liaison pour augmenter le poids moléculaire de cet agent de liaison et permettre ainsi une plus grande différence de poids moléculaire entre la substance marquée par une enzyme n'ayant pas réagi et les produits de la réaction formés au cours du dosage.

L'invention va être maintenant illustrée par des exemples de dosages immunoenzymatiques metant en oeuvre la Δ5,3 céto-stéroïde isomérase à titre d'enzyme pour le dosage par compétition d'une part de la progestérone et d'autre part de l'hormone lactogène placentaire humaine HLP, et pour le dosage direct de l'alphafoetoprotéine. Il faut toutefois noter que ces exemples ne sont pas limitatifs et que le procédé de dosage immunoenzymatique de l'invention-peut être utilisé pour un dosage quelconque nécessitant la séparation du conjugué substance protéinique/enzyme libre des complexes formés au cours du dosage.

Exemple

1/ Préparation des conjugués antigène-enzyme

On a utilisé le mode opératoire décrit dans la demande de brevet FR—A—2432713 (EP—A—7873).

La Δ5,3 céto-stéroïde isomérase a été thiolée par réaction avec l'anhydride S-acétyl mercaptosuccinique, à pH 7, puis dialyse et hydrolyse du groupement S-acétyle par l'hydroxylamine 0,1 M à pH 8; l'enzyme resultant a été ensuite dialysée.

a) Conjugué progestérone-Δ5,3 céto-stéroïde isomérase

On a préparé par ailleurs un dérivé de la progestérone, à savoir la 11α-bromoacétoxy-progestérone, en estérifiant de la 11α-hydroxyprogestérone par l'acide bromoacétique en présence de dicyclohexyl-carbodiimide. Le produit de réaction a été purifié par chromatographie sur silicagel.

On a fait ensuite réagir 10 mg de 11α-bromoacétoxyprogestérone avec 0,5 mg d'enzyme thiolée (6 thiols par mole d'enzyme) préparée selon le mode opératoire ci-dessus. La réaction a été réalisée pendant 16 heures à pH 8,2 et à la température ambiante. Après réaction, l'excès de stéroïde a été éliminé par filtration sur gel "Sephadex G 25". Deux moles de stéroïde ont réagi avec 1 mole d'enzyme. Selon le même mode opératoire, on a préparé un conjugué cortisol-Δ5,3-céto-stéroïde isomérase.

b) Conjugué HLP-Δ5,3-céto-stéroïde isomérase

On a thiolé la Δ5,3-céto-stéroïde isomérase selon le mode opératoire décrit ci-dessus. Le dérivé HLP à été préparé par une réaction d'échange de ponts disulfures en faisant réagir la HLP avec le 4-mercaptobutyrimidate de méthyle comme suit:

2 mg d'HLP sont dissous dans 300 μl d'eau amenée à pH 9,5 avec 100 μl de tampon carbonate-bicarbonate 0,1 N et additionnée de 1,3 mg de 4-mercaptobutyrimidate de méthyle.

On a fait ensuite réagir le dérive HLP préparé ci-dessus avec l'enzyme thiolée, activée par le dithio nitrobenzoate; après 16 heures de réaction l'excès de HLP a été éliminé par chromatographie d'affinité.

c) Conjugué anticorps anti-alphafoetoprotéine-Δ5,3-céto-stéroïde isomérase

Des anticorps anti-alphafoetoprotéine, (IgG) ont été mis à réagir avec de l'anhydride S-acétylmercaptosuccinique en utilisant le mode opératoire décrit précédemment pour la Δ5,3-céto-stéroïde isomérase. Les groupements S-acétyle ont été hydrolysés par l'hydroxylamine 0,1 M à pH 8.

Les IgG ainsi enrichies en groupements thiol ont été incubées une nuit avec de la Δ5,3-céto-stéroïde

6

# 0 022 005

isomérase, thiolée selon le mode opératoire décrit ci-dessus et activée par le dithio-nitrobenzoate. L'excès d'IgG a été éliminé par chromatographie sur Sépharose 4β-glutathion-oestradiol.

2/ Couplage du gel avec l'oestradiol 17β-glutathion

a) Préparation du dérivé oestradiol 17β-glutathion

Le 17β-bromoacétate d'oestradiol a été préparé par estérification de l'oestradiol (300 mg) à l'aide de l'acide bromoacétique (500 mg) en présence de 700 mg de dicyclohexylcarbodiimide, le milieu de réaction étant constitué par 2 ml de diméthylformamide ou de tétrahydrofurane. Après 16 heures de réaction, les produits ont été séparés sur colonne de silicagel.

60 mg de bromoacétate d'oestradiol-17β dissous dans 1 ml de diméthylformamide ont été incubés avec 300 mg de glutathion réduit dans 5 ml de tampon tris 0,1 M, pH 8,5 contenant 50% d'éthanol ou 50% de diméhylformamide. Après 16 heures de réaction, 20 ml d'eau ont été ajoutés et la phase aqueuse a été extraite deux fois par 50 ml d'éther. La phase aqueuse a ensuite été déposée sur une colonne de "Dowex" 1×2 (1,5×7 cm) équilibrée à pH 7. Après lavage avec 100 ml de tampon citrate 0,1 M, pH 6,5, puis 100 ml de tampon citrate 0,1 M, pH 2, la colonne a été éluée par 50 ml de tampon citrate 0,1 M, pH 2 contenant 50% d'éthanol. Le pH de l'éluat contenant le dérivé oestradiol-glutathion a été ajusté à pH 9.

b) Couplage gel-dérivé oestradiol

On a utilisé comme gel un gel de polyacrylamide-agarose (ULTROGEL Ac34 ou ULTROGEL Aç44) et on a couplé ce gel en faisant réagir 100 ml de gel avec 30 mg du dérivé oestradiol préparé ci-dessus selon le mode opératoire ci-après: 100 ml de gel ont été activés par 10 g de bromure de cyanogène à pH 11; après 5 minutes de réaction, le gel a été lavé par 1 litre d'eau et 200 ml de tampon bicarbonate de sodium 0,1 M pH 9. Le gel a été ajouté au dérivé glutathion oestradiol en solution dans 50 ml de tampon bicarbonate de sodium 0,1 M pH 9 contenant 50% d'éthanol. Après 16 heures de réaction le gel a été lavé par ce même tampon puis par 1 litre d'eau. La concentration en oestradiol dans la phase gel a été déterminée après hydrolyse par l'hydroxylamine M à pH 9 et dosage enzymatique de l'oestradiol libéré. La concentration en oestradiol du gel était de $7,5 \cdot 10^{-5}$ M.

3/ Dosage immunoenzymatique

a) Dosage par compétition

On a réalisé le dosage par compétition en utilisant des anticorps spécifiques de la progestérone ou de HLP et des anticorps anti γ-globuline, ces derniers permettant d'augmenter le poids moléculaire de l'anticorps spécifique. La réaction de compétition a été réalisée dans des tubes en polypropylène.

100 μl de la solution d'antigène à doser ont été incubés avec 100 μl d'anticorps spécifiques, 100 μl d'anticorps anti-γ globuline, 100 μl de la solution d'antigène-enzyme (0,05 UI). Après 1 heure de réaction, 500 μl de tampon ont été ajoutés et le contenu de chaque tube a été filtré sur microcolonnes (100 μl) du gel ULTROGEL oestradiol préparé selon le mode opératoire ci-dessus. Le nombre de microcolonnes était égal au nombre de tubes nécessaires aux dosages. L'activité enzymatique du filtrat a été déterminée par spectrophotométrie à 248 nm. Le gel a été ensuite régénéré par lavage à l'eau.

On a trouvé que, dans ces conditions, 95% du complexe anticorps/antigène-enzyme est récupéré après filtration sur gel, tandis que 98% du conjugué antigène-enzyme libre est retenu sur le gel.

On a répété le dosage ci-dessus en utilisant des quantités différentes d'antigènes à doser (HLP ou progestérone).

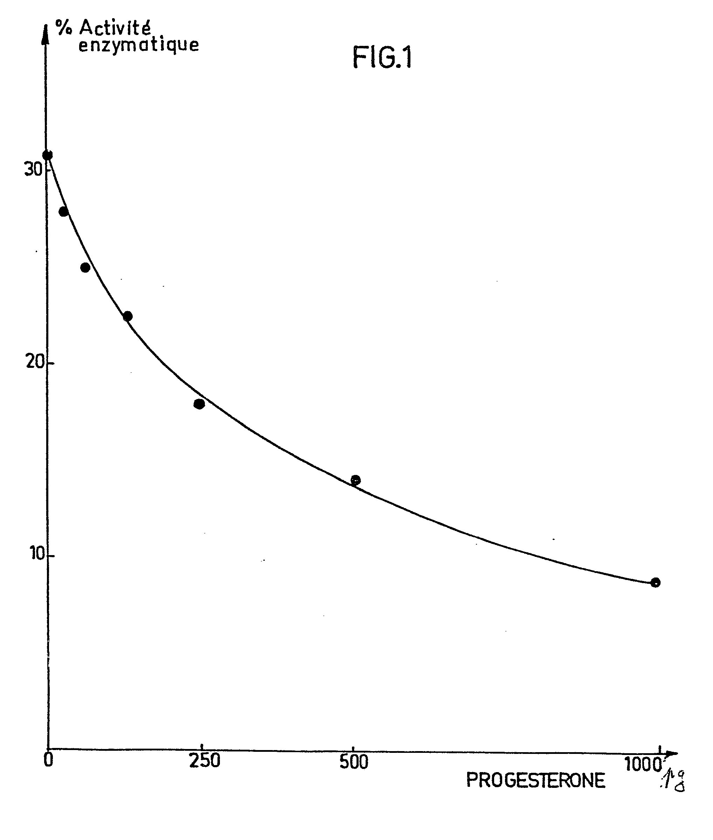

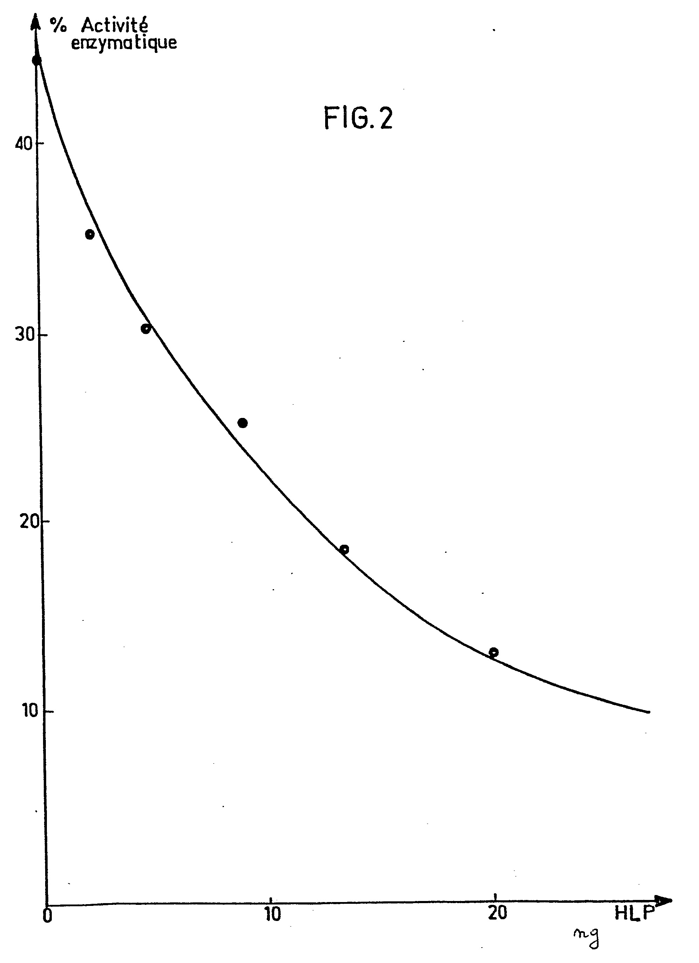

Après filtration, sur 0,1 ml de gel, de le solution obtenue après incubation, on a mesuré l'activité enzymatique de la solution ou filtrat. Les résultats obtenus sont indiqués sur les figures 1 et 2 annexées, la figure 1 étant relative à la progestérone et la figure 2 à l'hormone lactogène placentaire humaine HLP; sur ces figures, l'activité enzymatique, en %, est portée en ordonnées et le quantité d'antigène dosée est portée en abscisses. Ces résultats montrent que l'activité enzymatique du filtrat diminue à mesure que la quantité d'antigène augmente.

Ce procédé de dosage donne des résultats similaires aux autres méthodes de dosages (dosages radioimmunologiques ou dosages immunoenzymatiques utilisant un immunoadsorbant). Ces procédés sont plus rapides, puisqu'ils ne nécessitent qu'une filtration, et l'enzyme peut être déterminée directement sur des parties aliquotes de 200 μl. La quantité d'enzyme de 0;1 UI (unités internationales) correspond à 50 femtomoles de l'antigène; en général, 30 à 40% du conjugué antigène-enzyme est lié par les anticorps spécifiques en l'absence de l'antigène libre (c'est-à-dire de l'antigène à doser). Cette activité enzymatique, déterminée sur des parties aliquotes de 200 μl, varie de 200 à 40 milliunités d'adsorbance par minute. Cette forte activité enzymatique permet une bonne reproductibilité et on n'a pas noté d'interférence avec le sérum. En effet, seulement le sérum en fin de gestation est capable d'inhiber l'enzyme puisque la concentration en oestradiol est très élevée et peut inhiber l'enzyme, mais en fait les sérums à tester sont suffisamment dilués pour qu'il n'y ait pas d'interférence au cours du dosage.

b) Dosage direct: dosage de l'alphafoetoprotéine

50 μl de conjugué IgG-Δ5,3-céto-stéroïde isomérase (3 UI) préparé comme ci-dessus ont été incubés avec des quantités croissantes d'alpha foetoprotéine (0,1 à 10 ng dans 50 μl). Après 2 heures d'incubation à température ambiante, ces solutions ont été additionnées de 500 μl de tampon phosphate 0,1 M, et filtrées

7

# 0 022 005

sur 100 µl de gel (Ultrogel ACA-34-glutathion-oestradiol). Le conjugué isomérase-IgG en excès a été retenu sur la colonne (98%) alors que le complexe alpha foetoprotéine-IgG-isomérase de poids moléculaire élevé a été exclu du gel.

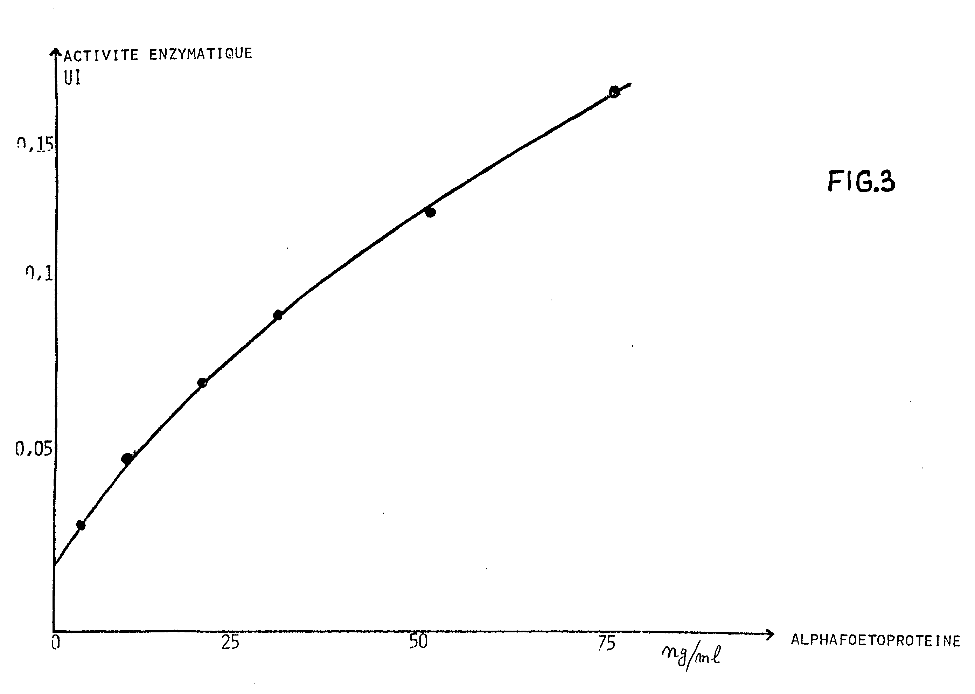

L'activité enzymatique du filtrat était proportionnelle à la quantité d'antigène présente dans le milieu d'incubation. Les résultats obtenus sont représentés sur la figure 3, sur laquelle on a porté en ordonnées l'activité enzymatique (UI) du filtrat, et en abscisses la quantité d'alphafoetoprotéine (ng/ml) dans l'échantillon à doser. L'activité enzymatique mesurée est fonction de la concentration en alpha foetoprotéine dans l'échantillon à doser.

TABLEAU I

Exemples de dosages enzymatiques

| Substance à doser | Substance marquée par une enzyme X*e | Agent de liaison | Produits de réaction | Type de dosage |
|---|---|---|---|---|
| | Ag*e | Ac? | Ag*e–Ac?; Ag*e | Direct de l'anticorps |
| Ag? | Ag*e | Ac | Ag*e–Ac<br>Ag?–Ac; Ag*e | Par compétition de l'antigène |
| | H*e | R? | H*e–R?; H*e | Dosage direct du récepteur |
| H? | H*e | R | H?–R<br>H*e–R; H*e | Dosage par compétition de l'hormone |
| | L*e | P? | L*e–P?; L*e | Dosage direct de la protéine de transport |
| L? | L*e | P | L*e–P<br>L?–P; L*e | Dosage par compétition du ligand |
| | Ac*e | Ag? | Ac*e–Ag?; Ac*e | Dosage direct de Ag |
| Ac? | Ac*e | Ag | Ac*e–Ag<br>Ac?–Ag; Ac*e | Dosage par compétition de Ac |

Légende:
Ag: Antigène
H: Hormone
R: Récepteur
X*e: Substance marqués par une enzyme (X=Ag, H, L, Ac...)
Ag?: Antigène à doser
H?, L? et Ac?: sont respectivement l'hormone, le ligand ou l'anticorps à doser.
Ac: Anticorps
L: Ligand
P: Protéine de transport

**Revendications**

1. Procédé de dosage enzymatique direct d'un agent de liaison par détermination du produit de la réaction de l'agent de liaison et de la substance marquée par une enzyme, ledit agent de liaison ayant une affinité vis-à-vis de la substance marquée par une enzyme consistant à

— faire réagir l'agent de liaison avec la substance marquée par l'enzyme pour obtenir une solution contenant un produit résultant de la réaction, de poids moléculaire supérieur à celui de la substance marquée par l'enzyme,

caractérisé en ce qu'ensuite on sépare la substance marquée par l'enzyme n'ayant pas réagi d'avec le ou les produits résultant de la réaction par une filtration de ladite solution sur un gel ayant des pores de taille suffisante pour exclure le ou les produits de la réaction, ce gel étant couplé avec un ligand pour lequel l'enzyme a une affinité, puis qu'on mesure l'activité enzymatique de la solution ainsi filtrée.

2. Procédé de dosage enzymatique par compétition d'une substance consistant à mettre en contact la solution contenant la substance avec cette substance marquée par une enzyme, et un agent de liaison qui a une certaine affinité pour la substance seule et pour la substance marquée par une enzyme, ce qui donne

8

# 0 022 005

une seconde solution dans laquelle se trouve le produit de la réaction de l'agent de liaison avec la substance qui était présente initialement et celui de la réaction avec la substance marquée par une enzyme, caractérisé en ce qu'ensuite on sépare la substance marquée par une enzyme n'ayant pas reagi d'avec les produits de réaction par une filtration de la seconde solution sur un gel ayant des pores de taille suffisante pour exclure le ou les produits de la réaction, ce gel étant couplé avec un ligand pour lequel l'enzyme a une affinité, puis qu'on mesure l'activité enzymatique de la solution ainsi filtrée.

3. Procédé de dosage enzymatique selon l'une des revendications 1 ou 2, caractérisé en ce que l'agent de liaison est choisi parmi les antigènes, les anticorps, les protéines de transport, les récepteurs hormonaux.

4. Procédé selon l'une des revendications précédentes, pour le dosage d'antigènes de nature stéroïdique ou peptidique, caractérisé en ce qu'il met en oeuvre un conjugué antigène-Δ5,3-céto-stéroïde isomérase comme substance marquée par une enzyme, en ce que le gel est couplé avec l'oestradiol 17β-glutathion, et en ce que ledit gel est choisi parmi les gels de polyacrylamide, d'agarose ou polyacrylamide-agarose.

5. Procédé selon la revendication 4, caractérisé en ce que l'antigène est la progestérone ou l'hormone lactogène placentaire humaine.

6. Procédé selon l'une des revendications 4 ou 5, caractérisé en ce que le gel est couplé avec l'oestradiol-17β-glutathion après activation du gel par le bromure de cyanogène.

7. Procédé selon la revendication 1 caractérisé en ce que la substance à doser est un antigène et en ce que le dosage est un dosage direct.

8. Procédé selon la revendication 7, caractérisé en ce que l'antigène est l'alpha foetoprotéine.

**Patentansprüche**

1. Verfahren zur direkten enzymatischen Bestimmung eines Bindemittels durch Bestimmung des Produkts der Reaktion des Bindemittels und der mit einem Enzym markierten Substanz, wobei das genannte Bindemittel eine Affinität zu der mit einem Enzym markierten Substanz hat, daraus bestehend, daß man das Bindemittel mit der mit dem Enzym markierten Substanz umsetzt, um eine Lösung zu erhalten, die ein aus der Reaktion resultierendes Produkt enthält, das ein höheres Molekulargewicht als die mit dem Enzym markierte Substanz hat, dadurch gekennzeichnet, daß man anschließend die mit dem Enzym markierte Substanz, die nicht reagiert hat von dem oder dan aus der Reaktion resultierenden Produkten durch eine Filtration der genannten Lösung auf einem Gel abtrennt, das Poren von genügender Weite aufweist, um das oder die Produkte der Reaktion auszuschließen, und das mit einem Liganden gekuppelt ist, zu dem das Enzym eine Affinität aufweist, und dann die enzymatische Aktivität der so filtrierten Lösung mißt.

2. Verfahren zur enzymatischen Bestimmung durch Konkurrenz einer Substanz, bestehend daraus, daß man die die Substanz enthaltende Lösung mit dieser mit einem Enzym markierten Substanz und einem Bindemittel, das eine gewisse Affinität zu der Substanz allein und zu der mit einem Enzym markierten Substanz aufweist, zusammenführt, was eine zweite Lösung ergibt, in der sich das Produkt der Reaktion des Bindemittels mit der Substanz, die anfangs vorhanden war, und dasjenige der Reaktion mit der mit einem Enzym markierten Substanz finden, dadurch gekennzeichnet, daß man anschließend die nicht umgesetzte, mit einem Enzym markierte Substanz von den Reaktions produkten durch eine Filtration der zweiten Lösung auf einem Gel abtrennt, das Poren von genügender Weite aufweist, um das oder die Produkte der Reaktion auszuschließen, und das mit einem Liganden gekuppelt ist, zu dem das Enzym eine Affinität aufweist, und dann die enzymatische Aktivität der so filtrierten Lösung mißt.

3. Verfahren zur enzymatischen Bestimmung nach einem der Ansprüche 1 oder 2, dadurch gekennzeichnet, daß das Bindemittel aus Antigenen, Antikörpern, Transportproteinen und Hormonrezeptoren ausgewählt ist.

4. Verfahren nach einem der vorstehenden Ansprüche zur Bestimmung von Antigenen von Steroid- oder Peptidnatur, dadurch gekennzeichnet, daß man ein Antigenkonjugat Δ5,3-Ketosteroidisomerase als mit einem Enzym markierte Substanz einsetzt, daß das Gel mit Östradiol-17β-glutathion gekuppelt ist und daß das genannte Gel aus Gelen von Polyacrylamid, Agarose oder Polyacrylamid-Agarose ausgewählt ist.

5. Verfahren nach Anspruch 4, dadurch gekennzeichnet, daß das Antigen Progesterin oder das menschliche laktogene Plazentahormon ist.

6. Verfahren nach einem der Ansprüche 4 oder 5, dadurch gekennzeichnet, daß das Gel nach Aktivierung des Gels mit Cyanbromid mit Östradiol-17β-glutathion gekuppelt ist.

7. Verfahren nach Anspruch 1, dadurch gekennzeichnet, daß die zu bestimmende Substanz ein Antigen ist, und daß die Bestimmung eine direkte Bestimmung ist.

8. Verfahren nach Anspruch 7, dadurch gekennzeichnet, daß das Antigen das α-Fötoprotein ist.

**Claims**

1. Process for direct enzymatic assay of a binding agent wherein the reaction product of said binding agent and the enzyme-labelled substance is determined, said binding agent having affinity for the enzyme-labelled substance, consisting of the reaction of the binding agent with the enzyme-labelled

9

# 0 022 005

substance to obtain a solution comprising the reaction product, having a molecular weight higher than that of the enzyme-labelled substance, characterized in that afterwards the unreacted enzyme-labelled substance is separated from the reaction product(s) by filtration of said solution on a gel having pores of size sufficient to exclude the reaction product(s), said gel being coupled to a ligand for which the enzyme has affinity, and the enzymatic activity of the filtered solution is measured.

2. Process for competitive enzymatic assay of a substance consisting of contacting the solution comprising the substance and same substance labelled with an enzyme and a binding agent having affinity for the substance and for the enzyme-labelled substance, to obtain a second solution comprising the reaction product of the binding agent and the starting substance and the reaction product of the binding agent and the enzyme-labelled substance characterized in that afterwards the unreacted enzyme-labelled substance is separated from the reaction products by filtrating the second solution on a gel having pores of size sufficient to exclude the reaction product(s), said gel being coupled to a ligand for which the enzyme has affinity, and the enzymatic activity of the filtered solution is measured.

3. Process for enzymatic assay according to claim 1 or 2, characterized in that the binding agent is selected from antigens, antibodies, carrier proteins, hormonal receptors.

4. Process according to any claim 1 to 3, for the determination of steroidal or peptidic antigens characterized in that an antigen Δ-5,3 keto steroid isomerase conjugate is used as the enzyme-labelled substance wherein the gel is coupled to 17β-glutathion oestradiol and wherein the gel is selected from polyacrylamide, agarose or polyacrylamide-agarose gels.

5. Process according to claim 4, characterized in that the antigen is progesterone or human placental lactogenic hormone.

6. Process according to claim 4 or 5, characterized in that the gel is coupled to 17β-glutathion oestradiol after gel activation with cyanogen bromide.

7. Process according to claim 1, characterized in that the substance to be assayed is an antigen and wherein the assay is direct.

8. Process according to claim 7, characterized in that the antigen is alpha foetoprotein.

10

FIG.1

FIG.2

ACTIVITE ENZYMATIQUE UI

FIG.3

ALPHAFOETOPROTEINE
ng/ml

0 022 005